# EUROPÄISCHE PATENTSCHRIFT

(11) **EP 3 150 278 B1**
(45) Veröffentlichungstag und Bekanntmachung des Hinweises auf die Patenterteilung: **07.08.2019**
(21) Anmeldenummer: 15002822.3
(22) Anmeldetag: 02.10.2015
(51) Int. Cl.: B01L 3/00, G01N 1/31, C12M 3/00, C12M 1/12

(54) **GEWEBEKASSETTE**
TISSUE CASSETTE
CASSETTE BIOLOGIQUE

(43) Veröffentlichungstag der Anmeldung: 05.04.2017
(73) Patentinhaber: Euroimmun Medizinische Labordiagnostika AG, 23560 Lübeck (DE)
(72) Erfinder: BUSCHTEZ, Michael, 23560 Lübeck (DE); ROTTMANN, Norbert, 23570 Lübeck (DE); KAYSER, Klaus, 69121 Heidelberg (DE)

(56) Entgegenhaltungen:
- EP-A1- 1 967 836
- EP-A2- 0 964 242
- EP-A2- 2 778 654
- WO-A1-00/19897
- US-A- 5 609 827
- Wolfgang Weyers: "Confusion-specimen mix-up in dermatopathology and measures to prevent and detect it", Dermatology Practical & Conceptual, 31 January 2014 (2014-01-31), XP055491929, DOI: 10.5826/dpc.0401a04

## Beschreibung

Die vorliegende Erfindung betrifft eine Gewebekassette umfassend eine aus Wänden und einem Boden ausgebildete äußere Schale mit einem Mittel zum Positionieren einer Gewebeprobe am Boden;
sowie ein Verfahren umfassend die Schritte Bereitstellen der Gewebekassette und Einbringen einer Gewebeprobe sowie Positionieren derselben am Boden.

Eine Vielzahl von medizinischen Diagnostizierverfahren kann man heutzutage auf Basis von Proben durchführen, die am Körper des Patienten gewonnen und nach einem Transport zentral in einem Labor untersucht werden.

Idealerweise gestattet die Beschaffenheit und Art der Probengewinnung, dass die Proben nach ihrer Ankunft im Labor nicht sofort abgearbeitet werden müssen. Das ist beispielsweise bei der Bestimmung von Elektrolyten in Blutproben der Fall. Die Qualität des Diagnoseergebnisses wird nicht dadurch beeinträchtigt, dass die Proben zunächst gesammelt werden, bis eine bestimmte Mindestzahl erreicht ist. Diese Mindestzahl erlaubt, dass eine Vielzahl von Proben parallel abgearbeitet werden kann, was die Organisation vereinfach und zum Einsparen von Ressourcen beiträgt.

Bei anderen Proben kann es erforderlich oder wenigstens vorteilhaft sein, sie nach der Gewinnung unverzüglich abzuarbeiten. Dies kann dadurch bedingt sein, dass die Probe vor der Abarbeitung nicht haltbar ist bzw. nicht eingefroren oder in sonstiger Weise konserviert werden kann, beispielsweise, weil dies die Qualität des Gewebes oder die Eigenschaften der darin enthaltenen Moleküle beeinträchtigen würde.

Im letzteren Fall wird die Probe, typischerweise eine Gewebeprobe, schnellstmöglich vom Ort der Gewinnung zum pathologischen Labor gebracht. Dort werden haltbare Gewebeschnitte angefertigt.

Die Gewebeprobe wird für den Transport typischerweise in ein spezielles Transportgefäß eingebracht, das im pathologischen Labor geöffnet wird. Das Gewebe wird anschließend in Paraffin eingebettet. Der entstehende Paraffinblock kann in Scheiben geschnitten werden, die sich für pathologische Färbungen und eine mikroskopische Untersuchung eignen.

Bei sämtlichen Arbeiten, auch in eiligen Fällen, ist eine stringente Identitätssicherung zu gewährleisten. Zum Schutz des Patienten ist unbedingt zu verhindern, dass Proben vertauscht und die Ergebnisse dem falschen Patienten zugeordnet werden. Ebenso muss sichergestellt werden, dass die Probe auch der richtigen Art von Untersuchung unterzogen wird.

Die Abnahme einer zweiten Probe, etwa weil die erste versehentlich auf einem falschen Parameter untersucht oder vertauscht wurde, ist aufgrund der damit verbundenen zusätzlichen Belastung des Patienten und des oft nur in begrenzter Menge vorhandenen Probenmaterials unbedingt zu vermeiden.

Die praktische Gewinnung und Verpackung der Probe wird nicht nur durch möglichen Zeitdruck erschwert, sondern dadurch, dass das an der Probengewinnung beteiligte Personal häufig in einer speziellen keimarmen Umgebung arbeitet und Handschuhe und Schutzkleidung trägt. In dieser Situation ist es besonders aufwändig, Transportgefäße neu vorzubereiten und zu beschriften.

Ein weiteres Problem besteht darin, dass die im Transportbehälter eingebrachte Gewebeprobe während des Transports eine Form oder Position einnehmen kann, welche die nachfolgende Prozessierung erschwert. Beispielsweise lässt sich ein Gewebestück, das in einer Ecke des Behälters verklebt ist, schwieriger paraffinieren und zu einem qualitativ hochwertigen Gewebeschnitt verarbeiten. Es besteht weiterhin die Gefahr, dass das Gewebestück während des Transportes in mehrere Stücke zerfällt.

Nicht zuletzt wird das Gewebestück bei der Gewinnung nach Möglichkeit in einer Orientierung im Transportgefäß eingebracht, die es dem Personal bei der nachfolgenden Abarbeitung erleichtert, besonders wichtige Teile der Probe, z.B. krankhaftes Gewebe, als solches zu erkennen und bei der Anfertigung der Gewebeschnitte besonders in den Vordergrund zu stellen. Eine Veränderung der Orientierung oder Position während des Transportes erschwert dies.

Vor diesem Hintergrund besteht eine der vorliegenden Erfindung zu Grunde liegende Aufgabe darin, eine Gewebekassette als Transportgefäß bereitzustellen, die dem Benutzer besonders flexibel gestattet, beispielsweise auch wenn er Handschuhe trägt, eine Gewebeprobe möglichst einfach, schnell und zuverlässig in der Gewebekassette zu positionieren.

Die Kassette soll möglichst einfach und robust ausgestaltet und insbesondere möglichst wenig beschädigungsanfällig sein.

Die Gewebekassette soll weiterhin eine möglichst bequeme, effiziente, sichere und hochwertige Abarbeitung, insbesondere Paraffinierung der darin transportierten Gewebeprobe, ermöglichen.

Die Gewebekassette soll eine ressourcensparende Abarbeitung der Gewebeprobe mit möglichst geringer Vermischung nacheinander eingesetzter Chemikalien gestatten.

Schließlich besteht eine weitere der Erfindung zu Grunde liegende Aufgabe darin, eine Gewebekassette bereitzustellen, die derartig ausgestaltet ist, dass der Benutzer ohne weitere Hilfsmittel, insbesondere ohne einen Stift in die Hand zu nehmen, beim Einbringen der Gewebeprobe Informationen übermitteln kann, beispielsweise zur Art der Probe, Identität des Patienten oder zur benötigten Untersuchung.

Das Dokument WO 00/19897 A1 beschreibt eine Gewebekassette mit Haken und Greifstiften zum Positionieren einer Gewebeprobe am Boden.

Das Dokument EP 1 967 836 A1 beschreibt eine Gewebekassette umfassend eine aus Wänden und einem Boden ausgebildete äußere Schale mit einem Mittel zum Positionieren einer Gewebeprobe am Boden.

Das Dokument EP 2 778 654 A2 beschreibt eine Gewebekassette umfassend eine aus Wänden und einem Boden ausgebildete äußere Schale. Hierbei kann ein Gel am Boden aufgebracht werden um als Haftschicht für eine Gewebeprobe zu dienen.

Das Dokument US 5,609,827 A beschreibt eine Gewebekassette umfassend eine aus Wänden und einem Boden ausgebildete äußere Schale und ein Mittel zum Positionieren einer Gewebeprobe am Boden.

Das Dokument EP 0 964 242 A2 beschreibt eine Gewebekassette mit einer Rippe als Markierung, wobei die Rippe die Innenfläche der Wände und/oder des Bodens der äußeren Schale in bestimmte Bereiche unterteilt.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Die der Erfindung zu Grunde liegende Aufgabe wird in einem ersten Aspekt gelöst durch eine erfindungsgemäße Gewebekassette umfassend eine aus Wänden und einem Boden ausgebildete äußere Schale mit einem Mittel zum Positionieren einer Gewebeprobe am Boden und eine Markierung an der Innenseite der Wand und/oder am Boden, wobei das Mittel zum Positionieren geeignet ist, um die Gewebeprobe nach dem gezielten Einbringen in die Gewebekassette an der erwünschten Position der Gewebekassette auf deren Boden zu fixieren,
wobei es sich bei dem Mittel zum Positionieren am Boden um eine haftende Schicht auf dem Boden handelt,
wobei es sich bei der haftenden Schicht um ein auf dem Boden der Kassette aufgetragenen Klebstoff handelt, der ausreichend haftet, um eine unbehandelte Gewebeprobe unverrückbar zu positionieren,
weiter umfassend eine innere Schale, die als Einsatz an die äußere Schale angepasst ist,
wobei die innere Schale einen flüssigkeitsdurchlässigen Boden aufweist und mit Hilfe eines Befestigungsmittels lösbar an der äußeren Schale befestigt werden kann.

Erfindungsgemäß ist die Gewebekassette so ausgestaltet, dass es sich bei dem Mittel zum Positionieren am Boden um eine haftende Schicht auf dem Boden handelt.

Vorzugsweise handelt es sich bei der haftenden Schicht um ein Klebekissen, bevorzugt bedeckt mit einer abziehbaren Abdeckfolie.

Vorzugsweise weist die äußere Schale eine Längsachse auf und die beiden Querwände bilden wenigstens eine Stufe.

Vorzugsweise wird die Stufe dadurch gebildet, dass der darüber liegende Teil der Querwand einen Winkel α1 von 15 bis 29° und der drunter liegende Teil der Querwand einen Winkel α2 von 30 bis 90° ausbildet.

Vorzugsweise liegt die Stufe auf einer vertikalen Höhe von wenigstens 30, bevorzugt wenigstens 40, noch bevorzugter wenigstens 50 % der Gesamthöhe der äußeren Schale.

Vorzugsweise bilden zusätzlich die Längswände eine Stufe, bevorzugt auf der gleichen Höhe wie die durch die Querwände gebildete Stufe, wobei die Längswände dadurch gekennzeichnet sind, dass der über der Stufe liegende Teil einen Winkel β1 ausbildet, der größer ist als der von den Querwänden über der Stufe ausgebildete Winkel α1, bevorzugt 45 bis 90°.

Erfindungsgemäß umfasst die Gewebekassette eine Markierung an der Innenseite der Wände und/oder am Boden.

Vorzugsweise unterteilt die Markierung die Innenfläche der Wände und/oder des Bodens der äußeren Schale in bestimmte Bereiche.

Vorzugsweise ist die Markierung ziffernblattartig angeordnet.

Erfindungsgemäß umfasst die Gewebekassette weiter eine innere Schale, die als Einsatz an die äußere Schale angepasst, wobei die innere Schale einen flüssigkeitsdurchlässigen Boden aufweist und mit Hilfe eines Befestigungsmittels lösbar an der äußeren Schale befestigt werden kann.

Vorzugsweise umfasst die Gewebekassette weiter eine Gewebeprobe, bevorzugter eine am Boden der äußeren Schale positionierte Gewebeprobe, bei der es sich noch bevorzugter um eine unbehandelte Gewebeprobe oder eine in Paraffin eingebettete Gewebeprobe handelt, am bevorzugtesten um eine unbehandelte Gewebeprobe.

Die der Erfindung zu Grunde liegende Aufgabe wird in einem zweiten Aspekt gelöst durch ein Verfahren umfassend die Schritte
a) Bereitstellen einer erfindungsgemäßen Gewebekassette, und
b) Einbringen einer Gewebeprobe sowie Positionieren derselben am Boden,
   wobei es sich bei der Gewebeprobe um eine unbehandelte Gewebeprobe handelt,
   weiter umfassend die Schritte
c) Einsetzen und Befestigen einer inneren Schale, die als Einsatz an die äußere Schale angepasst ist, wobei die innere Schale einen flüssigkeitsdurchlässigen Boden aufweist und mit Hilfe eines Befestigungsmittels lösbar an der äußeren Schale befestigt werden kann,
d) Einbetten der in der Gewebekassette befindlichen Gewebeprobe in Paraffin und
e) Loslösen und Entnehmen der inneren Schale mit der in Paraffin eingebetteten Gewebeprobe.

Vorzugsweise handelt es sich bei der Gewebeprobe um eine in Paraffin eingebettete Gewebeprobe. Die Gewebeprobe stammt bevorzugt von einem Säugetier, noch bevorzugter von einem Primaten, am bevorzugtesten von einem Menschen.

Die vorliegende Erfindung betrifft eine Gewebekassette, die ein Mittel zum Positionieren einer Probe umfasst. Als Mittel zum Positionieren ist jedes Mittel geeignet, welches die Gewebeprobe wenigstens nach dem Verschließen des Transportgefäßes bis zum Öffnen, bevorzugt vom Einbringen und Positionieren der Gewebeprobe im Transportgefäß bis einschließlich der Paraffinierung in einer gewünschten Position und Orientierung am Boden der äußeren Schale fixiert, optional relativ zu einer Markierung.

Die vorliegende Erfindung betrifft eine Gewebekassette, die für den Transport einer Gewebeprobe, bevorzugt einer beschädigungsanfälligen und/oder verderblichen Gewebeprobe geeignet ist. Die Probe stammt bevorzugt von Säugetieren, besonders bevorzugt von einem Menschen. Die Gewebekassette ist chemisch und physikalisch ausreichend widerstandsfähig für das Aufbereiten von Proben, beispielsweise für den Kontakt mit reaktiven Reagenzien wie organische Lösemitteln, das Arbeiten bei extremen Temperaturen und Verfahren wie das Zentrifugieren, insbesondere auch für das Paraffinieren einer darin befindlichen, positionierten Gewebeprobe. Geeignete Gefäße, bevorzugt aus Kunststoff, kann der Fachmann routinemäßig herstellen.

Die Gewebekassette umfasst ein Mittel zum Positionieren der Gewebeprobe. Unter "Mittel zum Positionieren" wird ein jegliches Mittel verstanden, das geeignet ist, um die Gewebeprobe nach dem gezielten Einbringen in die Gewebekassette, an der erwünschten Position der Gewebekassette auf deren Boden zu fixieren, insbesondere für den Transport und Paraffinierung. Beispielsweise kann es sich um eine haftende Schicht, um zum Aufspießen geeignete Haken handeln, oder die Probe wird durch den Deckel selbst und/oder eine Abdeckung zwischen Probe und Deckel unverrückbar auf dem Boden festgedrückt.

Bei der haftenden Schicht handelt es sich um ein auf den Boden der Gewebekassette aufgetragenen Klebstoff, der ausreichend haftet, um eine unbehandelte Gewebeprobe unverrückbar zu positionieren. Die Schicht kann durchgehend aufgetragen sein oder nicht durchgehend, beispielsweise in Form von Streifen oder gefüllten Kreisen.

Bei einem erfindungsgemäßen Klebekissen ist eine im Wesentlichen durchgehende Schicht Klebstoff auf dem Boden der Gewebekassette aufgetragen. Das Klebekissen kann durch Aufbringen eines beidseitig mit einer haftenden Schicht versehenen Klebestreifens auf den Boden bereitgestellt werden.

Die Klebefolie ist bevorzugt mit einer Lasche oder ähnlichen Verlängerung versehen, die es dem Bediener auch mit Handschuhen oder ähnlichen Behinderungen leicht erlaubt, sie abzuziehen und Zugang zum Klebekissen zu erhalten. Die Verlängerung kann bei einer unbenutzten Gewebekassette mit einer in die äußere Schale eingesetzten inneren Schale zunächst mit der inneren Schale verbunden sein, so dass die Klebefolie beim Herausnehmen der inneren Schale aus der äußeren Schale automatisch abgezogen wird.

Die erfindungsgemäße Gewebekassette weist bevorzugt eine Längsachse auf, an deren Enden sich zwei Querwände befinden. Besonders bevorzugt weisen die beiden Querwände wenigstens eine Stufe auf. Eine Stufe wird dadurch gebildet, dass zwei vertikal übereinander angeordnete Teilwände zur durch den Boden vorgegebenen Ebene unterschiedliche Winkel aufweisen.

Der Winkel, den die vertikal höher gelegene Teilwand einer Querwand mit der Bodenebene bildet, wird als α1 bezeichnet, der Winkel, den die vertikal darunter gelegene Teilwand mit der Bodenebene bildet, als α2. Bevorzugter ist α2 größer als α1, wobei α2 bevorzugter höchstens 90° beträgt. In einer bevorzugtesten Ausführungsform beträgt α1 15 bis 29° und α2 30 bis 90°. Parallel zur Längsachse weist die erfindungsgemäße Gewebekassette bevorzugt Längswände auf. Analog zu den Querwänden können diese wenigstens eine Stufe aufweisen, die dadurch gebildet wird, dass zwei vertikal übereinander angeordnete Teilwände zur durch den Boden vorgegebenen Ebene unterschiedliche Winkel aufweisen.

Der Winkel, den die vertikal höher gelegene Teilwand einer Längswand mit der Bodenebene bildet, wird als β1 bezeichnet, der Winkel, den die vertikal darunter gelegene Teilwand mit der Bodenebene bildet, als β2. Bevorzugter ist β1 größer als α1, wobei β1 bevorzugter größer als α1 ist und am bevorzugtesten größer als α1 ist und 45 bis 90° beträgt.

Die Gewebekassette weist eine Markierung an der Innenseite der Wände und/oder am Boden auf. Die erfindungsgemäße Markierung, insbesondere in Kombination mit dem Mittel zum Positionieren einer Gewebeprobe am Boden, bietet dem mit der Probengewinnung betrauten Personal die Möglichkeit, durch eine gezielte Positionierung der Probe am Boden Informationen zu übermitteln, ohne eine zusätzliche Kennzeichnung vorzunehmen.

Erfindungsgemäß kann eine erfindungsgemäße Gewebekassette eine bestimmte Anzahl von Markierungen aufweisen, die der Zahl der unterschiedlichen möglichen Untersuchungsverfahren entspricht. Wird ein Code vereinbart, nach dem eine bestimmte Markierung für ein bestimmtes Untersuchungsverfahren steht, oder bezeichnet die Markierung direkt ein solches Verfahren, so kann durch die bloße Positionierung der Probe nahe der Markierung angegeben werden. Eine zusätzliche Kennzeichnung, etwa handschriftlicher Art, ist nicht mehr erforderlich.

Eine bestimmte Positionierung der Probe relativ zur Markierung kann einen Hinweis auf bestimmte Teile der Probe geben, denen bei der Abarbeitung der Probe besondere Bedeutung zukommt, z.B. auf krankhaftes Gewebe, das bei den Gewebeschnitten besonders in den Vordergrund gestellt werden soll. Beispielsweise kann die Markierung ein Pfeil sein, der auf besonders zu beachtende Stelle der Gewebeprobe hinweist, beispielsweise die Seite, die krankhaftes Gewebe aufweist.

In einer bevorzugten Ausführungsform ist unter dem Begriff "Markierung", wie hierin verwendet, jegliche Art von Kennzeichnung zu verstehen, die geeignet ist, um Information zu übermitteln. In einer bevorzugten Ausführungsform handelt es sich um eine Beschriftung mit Nummer und/oder Buchstaben. Beispielsweise kann eine bestimmte Art von Untersuchung angegeben sein. In einer weiteren bevorzugten Ausführungsform kann es sich um eine räumliche Unterteilung handeln, wobei die erfindungsgemäße Gewebekassette in verschiedene Bereiche unterteilt wird. Diese Bereiche können ihrerseits wiederum wenigstens eine zusätzliche Markierung aufweisen, die eine Unterscheidung der Bereiche gestattet.

Bevorzugt ist die Markierung ziffernblattartig angeordnet, d.h. sie unterteilt die Fläche und den Boden der erfindungsgemäßen Gewebekassette dadurch in Bereiche, dass entlang einer an den Wänden der Gewebekassette verlaufenden Linie, die bevorzugt durch eine auf gleicher Höhe angeordneten Stufe gebildet ist, in bestimmten Abständen, bevorzugt regelmäßigen Abständen, Markierungen angebracht sind, beispielsweise Ziffern.

Verbindet man diese Markierungen im Geiste mit dem Mittelpunkt der erfindungsgemäßen Gewebekassette, welcher bei einer rechteckigen Gewebekassette durch den Schnittpunkt der beiden Diagonalen gebildet ist, so ergibt sich durch die Geraden zwischen den jeweiligen zwei Punktepaaren umfassend eine Markierung und den Mittelpunkt die Bereiche.

Erfindungsgemäß umfasst die erfindungsgemäße Gewebekassette nicht nur die äußere Schale, sondern weiter eine innere Schale, die als - bevorzugt nicht fest mit der äußeren Schale verbundener - Einsatz an die äußere Schale angepasst ist. Innere und äußere Schale sind bevorzugt so aneinander angepasst, dass eine in die äußere Schale eingebrachte Gewebeprobe zu einem Block umfassend die innere Schale und die Gewebeprobe paraffiniert wird, wenn Paraffin in die Gewebekassette umfassend äußere Schale, innere Schale und Gewebeprobe eingefüllt wird.

Ist eine innere Schale vorgesehen, so kann diese erfindungsgemäß unter Verwendung eines Befestigungsmittels mit der äußeren Schale fest, aber lösbar verbunden werden. Bei dem Befestigungsmittel kann es sich um jegliches Mittel handeln, dass zum Bewerkstelligen dieser lösbaren Verbindung geeignet ist.

In einer besonders bevorzugten Ausführungsform weist die äußere Schale entlang der Längsachse hervortretend an beiden Enden jeweils einen schienenartigen Vorsprung auf, wobei das Befestigungsmittel in Form einer Klammer ausgeführt ist, die an der äußeren Schale ansetzt und die innere Schale im verbundenen Zustand mit der äußeren Schale verbindet. Dabei weist das Befestigungsmittel zwei Aussparungen auf, die so angeordnet und an die beiden schienenartigen Vorsprünge an den Enden der äußeren Schale angepasst sind, dass jeweils ein Vorsprung eine Aussparung im verbundenen Zustand ausfüllt. Bevorzugt werden äußere und innere Schale in dieser Ausführungsform derart miteinander verbunden, dass das in Form einer Klammer ausgeführte Befestigungsmittel senkrecht zur Längsachse der äußeren Schale derart auf diese aufgesetzt wird, dass die schienenartigen Vorsprünge an beiden Enden der äußeren Schale in die beiden an sie angepassten Aussparungen eingeführt werden.

Bevorzugt umfasst die erfindungsgemäße Gewebekassette eine Gewebeprobe. Dabei kann es sich um eine paraffinierte Gewebeprobe handeln. Verfahren zur Paraffinierung von Gewebeproben sind im Stand der Technik beschrieben, beispielsweise in Lang (2006-2013) Histotechnik - Praxislehrbuch fuer die Biomedizinische Analytik. ISBN 978-3-7091-1189-5. In einer besonders bevorzugten Ausführungsform handelt es sich um eine unbehandelte Gewebeprobe. Unter "unbehandelt" ist bevorzugt zu verstehen, dass die Gewebeprobe so beschaffen ist, wie sie zum Transport versandt wird, einschließlich möglicher Behandlungen zur vorübergehenden Konservierung während des Transports.

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.

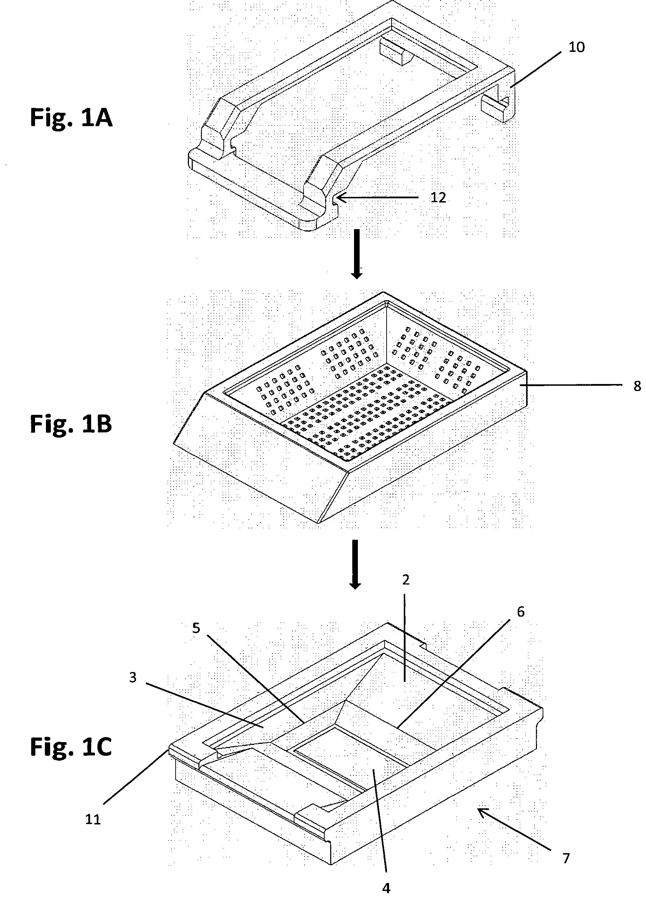

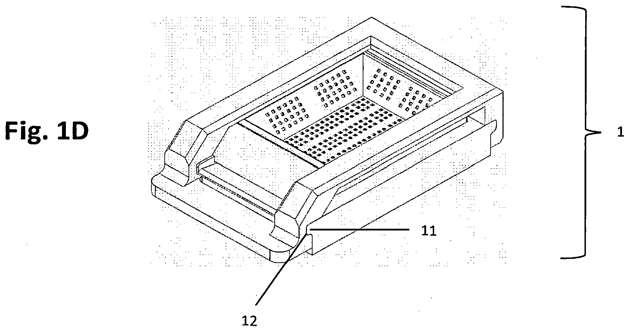

**Fig. 1** zeigt, wie erfindungsgemäß ein Befestigungsmittel (10), eine innere Schale (8) und eine äußere Schale (7) zu einer Gewebekassette (1) zusammengesetzt werden können, die alle drei Teile umfasst. Die äußere Schale weist eine Querwand (2) mit einer Stufe in der Querwand (6) und eine Längswand sowie eine Stufe in der Längswand (5) auf, zudem eine Klebekissen (4) am Boden. Die Befestigung wird dadurch erzielt, dass ein Vorsprung (11) an den Enden der äußeren Schale in an dessen Form angepasste Aussparungen (12) auf dem Befestigungsmittel hineingreift.

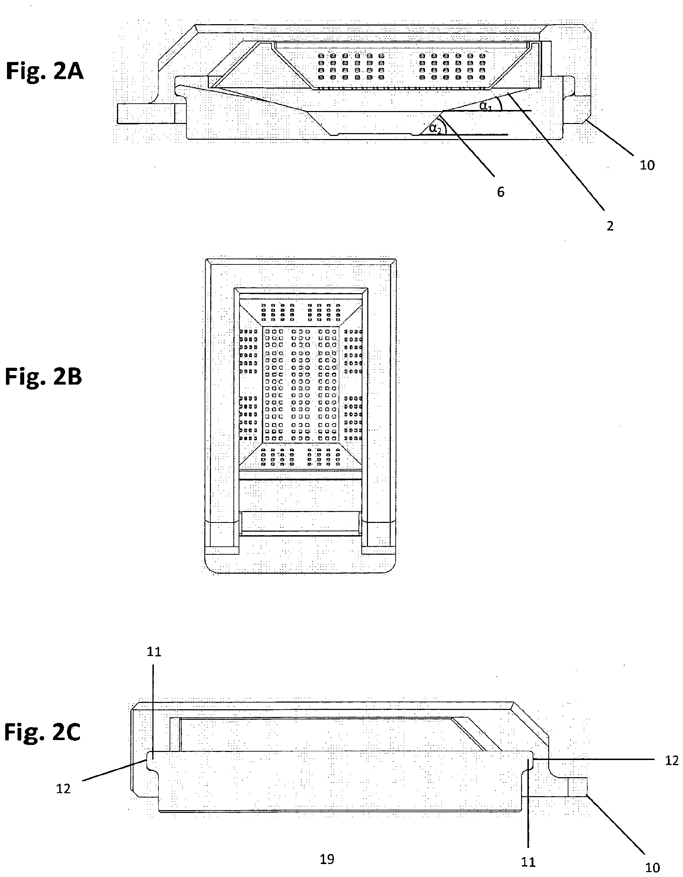

**Fig. 2** zeigt die gleiche erfindungsgemäße Gewebekassette umfassend Befestigungsmittel, innere Schale und äußere Schale wie in **Fig. 1** im Durschnitt entlang der Längsachse (**Fig. 2A**), von oben (**Fig. 2B**) und von der Außenseite auf die Längsachse blickend betrachtet (**Fig. 2C**). Die beiden Querwände aufweisen wenigstens eine Stufe, die dadurch gebildet wird, dass der darüber liegende Teil der Querwand einen Winkel α1 von 15 bis 29° und der darunter liegende Teil der Querwand einen Winkel α2 von 30 bis 90° ausbildet.

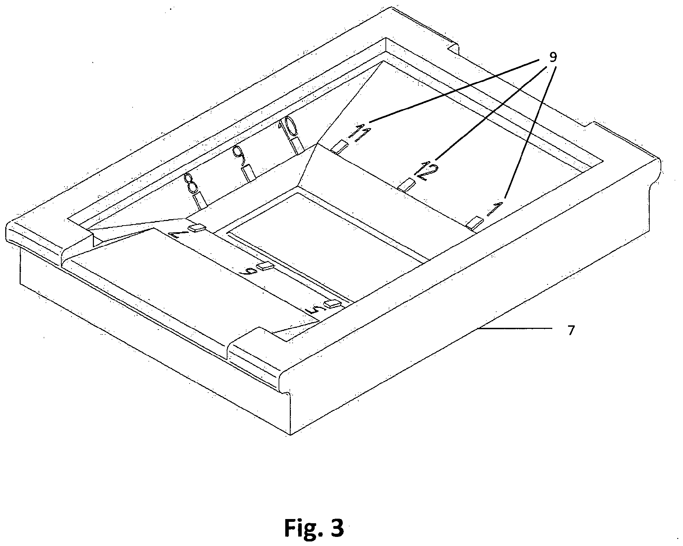

**Fig. 3** zeigt die äußere Schale der erfindungsgemäßen Gewebekassette. Die Markierungen (9) an der Innenfläche der Wände sind ziffernblattartig angeordnet. Durch Anordnung einer Gewebeprobe nahe oder relativ zu einer der Markierungen lässt sich durch deren bloße Position in der erfindungsgemäßen Gewebekassette Information übermitteln.

## Patentansprüche

1. Gewebekassette (1) umfassend eine aus Wänden und einem Boden ausgebildete äußere Schale (7) mit einem Mittel zum Positionieren einer Gewebeprobe am Boden und eine Markierung (9) an der Innenseite der Wand und/oder am Boden, wobei das Mittel zum Positionieren geeignet ist, um die Gewebeprobe nach dem gezielten Einbringen in die Gewebekassette an der erwünschten Position der Gewebekassette (1) auf deren Boden zu fixieren,
wobei es sich bei dem Mittel zum Positionieren am Boden um eine haftende Schicht auf dem Boden handelt,
wobei es sich bei der haftenden Schicht um ein auf dem Boden der Kassette aufgetragenen Klebstoff handelt, der ausreichend haftet, um eine unbehandelte Gewebeprobe unverrückbar zu positionieren,
weiter umfassend eine innere Schale (8), die als Einsatz an die äußere Schale (7) angepasst ist,
wobei die innere Schale (8) einen flüssigkeitsdurchlässigen Boden aufweist und mit Hilfe eines Befestigungsmittels lösbar an der äußeren Schale (7) befestigt werden kann.

2. Gewebekassette nach Anspruch 1,
wobei es sich bei der haftenden Schicht um ein Klebekissen (4) handelt, bedeckt mit einer abziehbaren Abdeckfolie, welche wiederum eine Lasche oder eine ähnliche Verlängerung aufweist.

3. Gewebekassette nach einem der Ansprüche 1 bis 2,
wobei die äußere Schale (7) eine Längsachse aufweist und die beiden Querwände wenigstens eine Stufe (6) aufweisen.

4. Gewebekassette nach Anspruch 3,
wobei die Stufe dadurch gebildet wird, dass der darüber liegende Teil der Querwand (2) einen Winkel α1 von 15 bis 29° und der drunter liegende Teil der Querwand (2) einen Winkel α2 von 30 bis 90° ausbildet.

5. Gewebekassette nach einem der Ansprüche 3 bis 4,
wobei die Stufe (6) auf einer vertikalen Höhe von wenigstens 30, bevorzugt wenigstens 40, noch bevorzugter wenigstens 50 % der Gesamthöhe der äußeren Schale (7) liegt.

6. Gewebekassette nach einem der Ansprüche 3 bis 5,
wobei zusätzlich die Längswände (3) eine Stufe (5) bilden,
bevorzugt auf der gleichen Höhe wie die durch die Querwände (2) gebildete Stufe (6),
wobei die Längswände (3) **dadurch gekennzeichnet sind, dass** der über der Stufe liegende Teil einen Winkel β1 ausbildet, der größer ist als der von den Querwänden (2) über der Stufe ausgebildete Winkel a1, bevorzugt 45 bis 90°.

7. Gewebekassette nach einem der Ansprüche 1 bis 6,
wobei die Markierung (9) die Innenfläche der Wände und/oder des Bodens der äußeren Schale (7) in bestimmte Bereiche unterteilt.

8. Gewebekassette nach einem der Ansprüche 1 bis 7,
wobei die Markierung (9) ziffernblattartig angeordnet ist.

9. Gewebekassette nach einem der Ansprüche 1 bis 8,
weiter umfassend eine Gewebeprobe, bevorzugter eine am Boden der äußeren Schale (7) positionierte Gewebeprobe, bei der es sich noch bevorzugter eine unbehandelte Gewebeprobe oder eine in Paraffin eingebettete Gewebeprobe handelt, am bevorzugtesten um eine unbehandelte Gewebeprobe.

10. Verfahren umfassend die Schritte
a) Bereitstellen einer Gewebekassette (1) nach einem der Ansprüche 1 bis 9, und
b) Einbringen einer Gewebeprobe sowie Positionieren derselben am Boden,
wobei es sich bei der Gewebeprobe um eine unbehandelte Gewebeprobe handelt,
weiter umfassend die Schritte
c) Einsetzen und Befestigen einer inneren Schale (8), die als Einsatz an die äußere Schale (7) angepasst ist, wobei die innere Schale (8) einen flüssigkeitsdurchlässigen Boden aufweist und mit Hilfe eines Befestigungsmittels lösbar an der äußeren Schale (7) befestigt werden kann,
d) Einbetten der in der Gewebekassette (1) befindlichen Gewebeprobe in Paraffin und
e) Loslösen und Entnehmen der inneren Schale (8) mit der in Paraffin eingebetteten Gewebeprobe.

## Claims

1. Tissue cassette (1) comprising an outer shell (7) which is formed of walls and a base and which has a means for positioning a tissue sample on the base, and a marking (9) on the inner face of the wall and/or on the base, wherein the positioning means is suitable for fixing the tissue sample, after targeted introduction thereof into the tissue cassette, at the desired position on the base of the tissue cassette (1),
wherein the means for positioning on the base is in the form of an adhesive layer on the base,
wherein the adhesive layer is in the form of a glue which is applied to the base of the cassette and which is sufficiently adhesive to position an untreated tissue sample immovably,
further comprising an inner shell (8), which is adapted as insert to the outer shell (7),
wherein the inner shell (8) has a liquid-permeable base and can be secured releasably on the outer shell (7) with the aid of a securing means.

2. Tissue cassette according to Claim 1,
wherein the adhesive layer is in the form of an adhesive pad (4), covered by a tear-off cover film which in turn has a tab or a similar extension.

3. Tissue cassette according to either of Claims 1 and 2,
wherein the outer shell (7) has a longitudinal axis, and the two transverse walls have at least one step (6).

4. Tissue cassette according to Claim 3,
wherein the step is formed by the fact that the part of the transverse wall (2) lying above it forms an angle α1 of 15 to 29° and the part of the transverse wall (2) lying below it forms an angle α2 of 30 to 90°.

5. Tissue cassette according to either of Claims 3 and 4,
wherein the step (6) lies at a vertical height of at least 30, preferably at least 40, more preferably at least 50% of the total height of the outer shell (7).

6. Tissue cassette according to one of Claims 3 to 5,
wherein the longitudinal walls (3) additionally form a step (5),
preferably at the same height as the step (6) formed by the transverse walls (2),
wherein the longitudinal walls (3) are **characterized in that** the part lying above the step forms an angle β1, which is greater than the angle α1 formed by the transverse walls (2) above the step, preferably 45 to 90°.

7. Tissue cassette according to one of Claims 1 to 6,
wherein the marking (9) divides the inner face of the walls and/or of the base of the outer shell (7) into defined regions.

8. Tissue cassette according to one of Claims 1 to 7,
wherein the marking (9) is arranged like a clock face.

9. Tissue cassette according to one of Claims 1 to 8,
further comprising a tissue sample, preferably a tissue sample positioned on the base of the outer shell (7), more preferably an untreated tissue sample or a paraffin-embedded tissue sample, most preferably an untreated tissue sample.

10. Method comprising the steps of
a) making available a tissue cassette (1) according to one of Claims 1 to 9, and
b) introducing a tissue sample and positioning same on the base,
wherein the tissue sample is an untreated tissue sample,
further comprising the steps of
c) inserting and securing an inner shell (8), which is adapted as insert to the outer shell (7), wherein the inner shell (8) has a liquid-permeable base and can be secured releasably on the outer shell (7) with the aid of a securing means,
d) embedding the tissue sample, located in the tissue cassette (1), in paraffin, and
e) detaching and removing the inner shell (8) with the paraffin-embedded tissue sample.

## Revendications

1. Cassette biologique (1) comportant une coque extérieure (7) formée à partir de parois et d'un fond et dotée d'un moyen servant au positionnement d'un échantillon de tissu sur le fond et un marquage (9) sur le côté intérieur de la paroi et/ou sur le fond, dans laquelle le moyen servant au positionnement se prête à fixer l'échantillon de tissu sur le fond de la cassette biologique à la position souhaitée de la cassette biologique (1) après l'introduction ciblée dans la cassette biologique,
dans laquelle le moyen servant au positionnement sur le fond est une couche adhésive sur le fond,
dans laquelle la couche adhésive est un adhésif appliqué sur le fond de la cassette, lequel adhère suffisamment pour positionner de manière immobile un échantillon de tissu non traité,
comportant en outre une coque intérieure (8) qui est adaptée à la coque extérieure (7) en tant qu'insert,
dans laquelle la coque intérieure (8) comprend un fond perméable aux liquides et peut être fixée de manière amovible à la coque extérieure (7) à l'aide d'un moyen de fixation.

2. Cassette biologique selon la revendication 1,
dans laquelle la couche adhésive est un tampon adhésif (4), recouvert d'une feuille de recouvrement pelable, laquelle comprend pour sa part une languette ou un prolongement similaire.

3. Cassette biologique selon la revendication 1 ou 2,
dans laquelle la coque extérieure (7) présente un axe longitudinal et les deux parois transversales comprennent au moins un gradin (6).

4. Cassette biologique selon la revendication 3,
dans laquelle le gradin est formé de telle sorte que la partie de la paroi transversale (2) située au-dessus de celui-ci forme un angle α1 de 15 à 29° et la partie de la paroi transversale (2) située en dessous de celui-ci forme un angle α2 de 30 à 90°.

5. Cassette biologique selon la revendication 3 ou 4,
dans laquelle le gradin (6) se situe à une hauteur verticale d'au moins 30, de préférence d'au moins 40, de manière plus préférée d'au moins 50 % de la hauteur totale de la coque extérieure (7).

6. Cassette biologique selon l'une des revendications 3 à 5,
dans laquelle les parois longitudinales (3) forment en outre un gradin (5),
de préférence à la même hauteur que le gradin (6) formé par les parois transversales (2),
dans laquelle les parois longitudinales (3) sont **caractérisées en ce que** la partie située au-dessus du gradin forme un angle β1 qui est supérieur à l'angle α1 formé par les parois transversales (2) au-dessus du gradin, de préférence de 45 à 90°.

7. Cassette biologique selon l'une des revendications 1 à 6,
dans laquelle le marquage (9) divise la surface intérieure des parois et/ou du fond de la coque extérieure (7) en régions déterminées.

8. Cassette biologique selon l'une des revendications 1 à 7,
dans laquelle le marquage (9) est disposé en forme de cadran.

9. Cassette biologique selon l'une des revendications 1 à 8,
comportant en outre un échantillon de tissu, de préférence un échantillon de tissu positionné sur le fond de la coque extérieure (7), dans laquelle il s'agit de manière plus préférée d'un échantillon de tissu non traité ou d'un échantillon de tissu inclus dans de la paraffine, mieux encore d'un échantillon de tissu non traité.

10. Procédé comprenant les étapes consistant à
a) fournir une cassette biologique (1) selon l'une des revendications 1 à 9 et
b) introduire un échantillon de tissu et positionner celui-ci sur le fond, dans laquelle l'échantillon de tissu est un échantillon de tissu non traité, comportant en outre les étapes consistant à
c) insérer et fixer une coque intérieure (8) qui est adaptée à la coque extérieure (7) en tant qu'insert, dans lequel la coque intérieure (8) comprend un fond perméable aux liquides et peut être fixée de manière amovible à la coque extérieure (7) à l'aide d'un moyen de fixation,
d) inclure l'échantillon de tissu se trouvant dans la cassette biologique (1) dans de la paraffine et
e) détacher et retirer la coque intérieure (8) dotée de l'échantillon de tissu inclus dans de la paraffine.
